# EUROPEAN PATENT APPLICATION

(11) **EP 1 495 711 A1**
(43) Date of publication of application: **12.01.2005**
(21) Application number: 04016015.2
(22) Date of filing: 07.07.2004
(51) Int. Cl.: A61B 1/00, A61L 29/00

(54) **Flexible tube for an endoscope and an endoscope equipped with the flexible tube**

(30) Priority: 07.07.2003 JP 2003193086
(71) Applicant: PENTAX Corporation, Tokyo 174-8639 (JP)
(72) Inventor: Hosoi, Masayoshi, Tokyo 174-8639 (JP); Shijo, Yoshihisa, Tokyo 174-8639 (JP)
(74) Representative: Schaumburg, Thoenes, Thurn, Landskron Patentanwälte

(57) **Abstract**

A flexible tube for an endoscope includes a tubular core member, and an outer cover provided around the core member, the outer cover having a laminated structure comprised of a first layer and a second layer which forms an outermost layer of the laminated structure. The first layer is formed of a material containing as a major component thereof polyolefin-based thermoplastic elastomer or styrene-ethylene · butylene-styrene block copolymer, and the second layer is formed of a material containing as a major component thereof polyolefin. The first and second layers are arranged adjacent to each other. The constituent material of the second layer may further contain thermoplastic elastomer.

## Description

### BACKGROUND OF THE INVENTION

### Field of the Invention

The present invention relates to a flexible tube for an endoscope and an endoscope equipped with the flexible tube.

### Description of the Prior Art

In an endoscopic examination, a flexible insertion tube of an endoscope is inserted along the body cavity to a deep part of a patient such as the stomach, duodenum, small intestine, and large intestine. For this reason, the flexible insertion tube of the endoscope is provided with an outer cover to improve ease of the inserting operation (that is, flexibility), which reduces a burden on a patient. In addition, the outer cover prevents fluids such as body fluids from entering the interior of the endoscope. In the prior art, an elastic material such as urethane-based elastomer has been generally used as a constituent material of the outer cover of the flexible insertion tube (see Japanese Patent Publication No. Hei 7-110270 (page 1, right column, lines 2-8), for example).

In the meantime, since an endoscope is repeatedly used, it must be cleaned and disinfected after each use. However, the above-mentioned material which has been conventionally used has a problem in that heat resistance and chemical resistance thereof are poor. For this reason, repeated cleaning, sterilization and disinfection of the endoscope deteriorates the outer cover of the flexible insertion tube, and then the flexibility of the outer cover itself is lowered, which may result in a case that it becomes difficult to insert the flexible insertion tube into a tubular cavity. Further, in a case where such deterioration is severe, small cracks and the like will occur, and as a result, there is a case that the constituent material of the outer cover of the flexible insertion tube will peel off.

### SUMMARY OF THE INVENTION

It is therefore an object of the present invention to provide a flexible tube for an endoscope having excellent heat resistance, chemical resistance and abrasion resistance with sufficient flexibility (elasticity), and an endoscope equipped with such a flexible tube.

In order to achieve the object, the present invention is directed to a flexible tube for an endoscope, comprising a tubular core member, and an outer cover provided around the core member. The outer cover has a laminated structure comprised of a first layer and a second layer which forms an outermost layer of the laminated structure. The first layer is formed of a material containing as a major component thereof polyolefin-based thermoplastic elastomer or styrene-ethylene butylene-styrene block copolymer, and the second layer is formed of a material containing as a major component thereof polyolefin.

According to the present invention described above, it is possible to provide a flexible tube for an endoscope having excellent heat resistance, chemical resistance and abrasion resistance with maintaining sufficient flexibility.

In the present invention, it is preferred that the first layer and the second layer are arranged adjacent to each other. This makes it possible to provide a more stable outer cover of a flexible tube with maintaining the excellent heat resistance, chemical resistance and abrasion resistance.

Further, in the present invention, it is also preferred that the material of the second layer further contains thermoplastic elastomer. This makes it possible to impart more sufficient flexibility to the flexible tube with maintaining the excellent heat resistance, chemical resistance and abrasion resistance.

In this case, it is preferred that the amount of the thermoplastic elastomer contained in the material of the second layer is equal to or less than 70 parts by weight with respect to 100 parts by weight of the polyolefin. This also makes it possible to impart more sufficient flexibility to the flexible tube with maintaining the excellent heat resistance, chemical resistance and abrasion resistance.

Further, in the present invention, it is preferred that the average thickness of the second layer is in the range of 0.01 to 0.5mm. This makes it possible to provide a flexible tube for an endoscope having more excellent abrasion resistance with maintaining the sufficient flexibility.

Furthermore, in present invention, it is also preferred that the polyolefin is mainly constituted from polypropylene. This makes it possible to provide a flexible tube for an endoscope having more excellent heat resistance with maintaining the sufficient flexibility.

Moreover, in present invention, it is also preferred that the amount of the polyolefin-based thermoplastic elastomer or styrene-ethylene · butylene-styrene block copolymer contained in the material of the first layer is 50wt% or more. This makes it possible to provide a flexible tube for an endoscope which can exhibit more excellent chemical resistance and heat resistance.

In this case, it is also preferred that the material of the first layer contains both the polyolefin-based thermoplastic elastomer and the styrene-ethylene butylene-styrene block copolymer, wherein the amount of the polyolefin-based thermoplastic elastomer and the styrene-ethylene butylene-styrene block copolymer contained in the material of the first layer is 50wt% or more. This also makes it possible to provide a flexible tube for an endoscope which can exhibit more excellent chemical resistance and heat resistance.

Moreover, in the present invention, it is also preferred that the average thickness of the outer cover is in the range of 0.08 to 0.9mm. This makes it possible to protect the core member and elongated members arrange inside the core member from fluids such as body fluids.

Moreover, in the present invention, it is also preferred that when the average thickness of the first layer is defined as a (mm), and the average thickness of the second layer is defined as b (mm) , the relationship represented by the equation of 0.25 ≤ b/a ≤ 1.0 is satisfied. This makes it possible to provide a flexible tube for an endoscope having more excellent chemical resistance, heat resistance and abrasion resistance with maintaining sufficient flexibility.

Another aspect of the present invention is directed to an endoscope equipped with the flexible tube as described above.

These and other objects, structures and results of the present invention will be apparent more clearly when the following detailed description of the preferred embodiments is considered taken in conjunction with the appended drawings.

### BRIEF DESCRIPTION OF THE DRAWINGS

Fig. 1 is an overall view showing the embodiment of an electronic endoscope (electronic scope) to which the endoscope according to the present invention is applied.
Fig. 2 is an enlarged longitudinal sectional view of a portion of a flexible tube of the electronic endoscope shown in Fig. 1.
Fig. 3 is an enlarged cross-sectional view of another embodiment of the flexible tube of the endoscope of the present invention.

### DETAILED DESCRIPTION OF THE INVENTION

Hereinafter, a detailed description of the preferred embodiments of a flexible tube for an endoscope and an endoscope equipped with the flexible tube according to the present invention will be given with reference to the appended drawings.

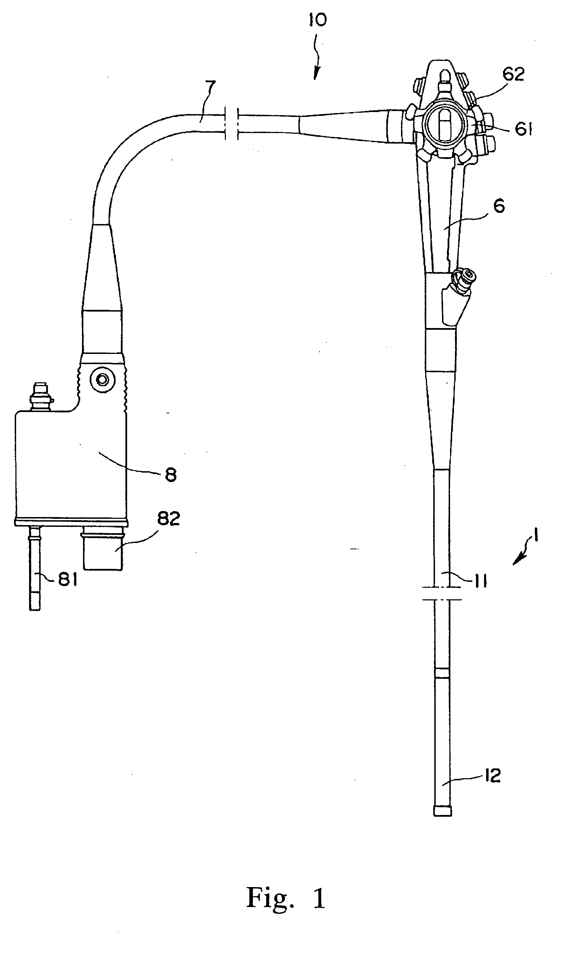

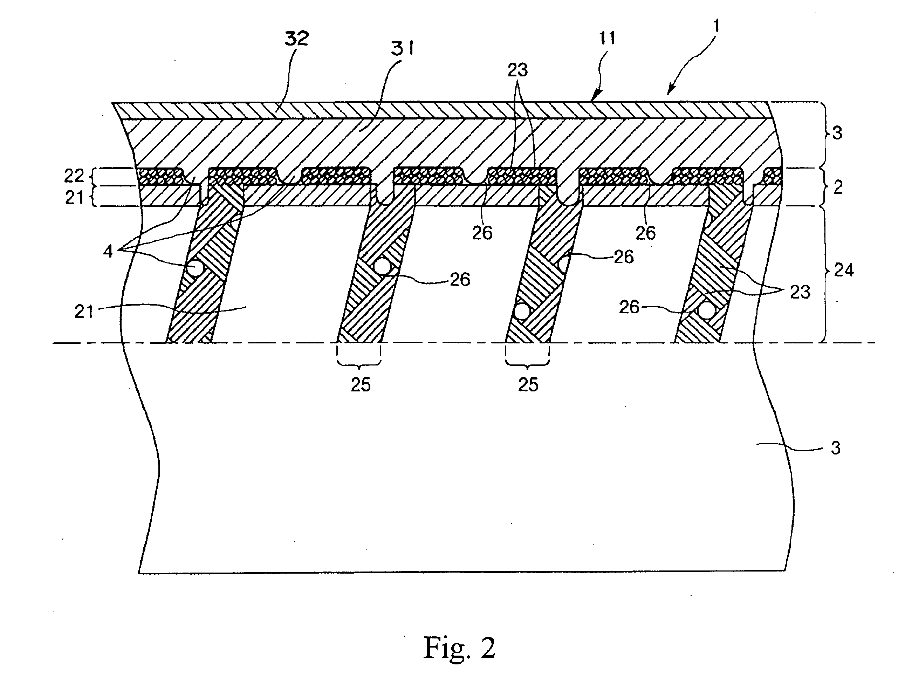

Fig. 1 is an overall view showing the embodiment of an electronic endoscope (electronic scope) to which the endoscope according to the present invention is applied, and Fig. 2 is an enlarged longitudinal sectional view of a portion of a flexible tube of the electronic endoscope shown in Fig. 1. In the following description, the upper side and the lower side in Fig. 1 will be referred to as "base or proximal end" and "tip or distal end", respectively.

As shown in Fig. 1, an electronic endoscope 10 includes an elongated flexible insertion tube 1 having flexibility, an operating section 6 provided on the base end of the flexible insertion tube 1, which is held by an operator to manipulate the electronic endoscope 10, a flexible connection tube 7 connected at the one end thereof to the operating section 6, and a light source plug section 8 provided on the other end of the flexible connection tube 7. The flexible insertion tube 1 is constructed from a main flexible tube section 11 and a bendable tube section 12 provided at the tip of the main flexible tube section 11.

Each of the flexible insertion tube 1 and the flexible connection tube 7 is manufactured using a flexible tube for an endoscope of the present invention which is obtained by covering the outer periphery of a (tubular) core member having a hollow space with an outer cover 3.

On one side surface of the operating section 6, there are provided operating knobs 61 and 62. When changing the direction of the bendable tube section 12, the operator turns each of the operating knobs 61 and 62 to pull appropriately wires (not shown) arranged inside the flexible insertion tube 1. In this way, the bendable tube section 12 can be bent to a desired direction.

An imaging element (CCD) not shown in the drawings is provided inside the tip end portion of the bendable tube section 12 to take observation images of an observation region. Further, an image signal connector 82 is provided at the tip end portion of the light source plug section 8. The image signal connector 82 is connected to a light source processor (not shown in the drawings) which is connected to a monitor (not shown in the drawings) via a cable. Furthermore, a light source connector 81 is provided at the tip end portion of the light source plug section 8, and this light source connector 81 is connected to the light source processor.

Light emitted from the light source processor passes through the light source connector 81 and a light guide (not shown in the drawings) that runs inside the light source plug section 8, the flexible connection tube 7, the operating section 6, and the flexible insertion tube 1, and then the light is irradiated from the tip end portion of the bendable tube section 12 (that is, the tip end of the flexible insertion tube 1) toward the observation region for illumination. Such a light guide is composed of a bundle of optical fibers made of quartz, multicomponent glass, plastic or the like, for example.

The reflected light from the observation region illuminated by the illumination light (observation image) is received by the imaging element. Then, the imaging element outputs an image signal corresponding to the observation image taken by the imaging element. The image signal is transmitted to the light source plug section 8 via an image signal cable (not shown in the drawings) which extends inside the flexible insertion tube 1, the operating section 6, and the flexible connection tube 7 in order to connect the imaging element and the image signal connector 82.

Then, in the light source plug section 8 and the light source processor, the image signal is subjected to predetermined processing (such as signal processing, image processing, and the like) , and then the processed signal is sent to the monitor. In this way, an image (electronic image) taken by the imaging element is displayed on the screen of the monitor in the form of a motion picture.

### <Flexible insertion tube 1>

As shown in Fig. 2, the flexible insertion tube 1 is constructed from the main flexible tube section 11 and the bendable tube section 12 provided at the tip of the main flexible tube section 11. The flexible insertion tube 1 has a core member 2 and the outer cover 3 that covers the outer periphery of the core member 2 (that is, the outer cover 3 is provided around the core member 2). Inside the flexible insertion tube 1 (that is, inside the core member 2), there are provided hollow spaces 24 through which elongated members such as optical fibers, electrical cables, wires, tubes and the like (which are omitted from the drawing) can be passed.

The core member 2 for the main flexible tube section 11 is constructed from a spiral coil 21 and a reticular tube (braided member) 22 which covers the outer periphery of the spiral coil 21. Further, the core member 2 for the bendable tube section 12 is constructed from a plurality of nodal rings (not shown in the drawings) rotatably coupled with each other and a reticular tube which covers the outer periphery of the nodal rings. In this way, the core member 2 is formed into an elongated tubular shape.

The spiral coil 21 is formed by winding a band-shaped member in a helical or spiral form with a gap 25 between the adjacent windings. Further, the spiral coil 21 is formed so as to have a substantially uniform internal diameter along the entire length thereof. Preferred examples of a material to be used for the spiral coil 21 and the nodal ring include stainless steel, copper alloys, and the like.

The reticular tube (such as the reticular tube 22) is formed by braiding a plurality of fine wire bundles in which each bundle includes metal or nonmetal fine wires 23 arranged side by side. Preferred examples of a material to be used for the fine wire 23 include stainless steel, copper alloys, and the like. Further, at least one of the fine wires 23 constituting the reticular tube may be covered with a resin material.

The reticular tube 22 has spaces (openings) 26 due to the stitches of the braided fine wires 23. These spaces 26 form concave portions at the positions that overlap with the outer periphery of the spiral coil 21, and form holes extending to the hollow spaces 24 at the positions that overlap with the gaps 25 of the spiral coil 21. Therefore, a plurality of holes and concave portions are formed in the outer periphery of the core member 2.

In the interior space of the core member 2 (that is, inside the flexible insertion tube 1), a solid lubricant such as molybdenum disulfide, boron nitride (BN), polytetrafluoroethylene (fluorine-based resin), graphite or fluorocarbon ((CF)ₙ) is provided. The solid lubricant is provided around the elongated members described above. This makes it possible to decrease sliding resistance (frictional resistance) between the elongated members or between each of the elongated members and the core member 2. Therefore, when the flexible insertion tube 1 (the main flexible tube section 11 and the bendable tube section 12) is bent, each of the elongated members smoothly moves in the longitudinal direction (axis direction) of the core member 2, so that the bending resistance thereof becomes small. Further, tension or pressure on the optical fibers constituting the light guide, or buckling of these optical fibers is suppressed, and as a result, damage or fracture of the light guide can be effectively prevented.

The outer periphery of the core member 2 is covered with the outer cover 3. The outer cover 3 has the function of preventing body fluids or the like from entering the interior of the flexible insertion tube 1, because the outer cover 3 directly comes into contact with the inner wall of a tubular organ such as the alimentary canal.

A plurality of protruding portions (anchors) 4 are integrally formed on the inner surface of the outer cover 3. These protruding portions protrude toward the inside so as to extend into the spaces formed in the outer periphery of the core member 2. Specifically, these protruding portions 4 extend into the plurality of holes and concave portions formed in the outer periphery of the core member 2. The tips of the protruding portions 4 that protrude into the concave portions are formed so as to reach the outer periphery of the spiral coil 21. The protruding portions 4 that protrude into the holes are formed to be even longer so that the tips thereof can be extended into the gaps 25 of the spiral coil 21.

By forming these protruding portions 4, an anchoring effect can be obtained, and this provides reliable securing between the outer cover 3 and the core member 2. As a result, even in a case where the flexible insertion tube 1 is bent, the outer cover 3 will maintain an adhering state with the core member 2, and will undergo large expansion and contraction to follow the bending of the core member 2. Further, the restoring force of the outer cover 3 undergoing such large expansion and contraction is strong enough to serve as a force for restoring the shape of the bent flexible insertion tube 1.

Further, due to the protruding portions 4 described above, the outer cover 3 firmly adheres to the reticular tube 22, so that the outer cover 3 will be difficult to peel off from the reticular tube 22 even over repeated use. Accordingly, the flexible insertion tube 1 will have excellent durability.

In a case where at least one of the fine wires 23 constituting the reticular tube 22 is covered with a resin material, at least a part of the resin material (covering layer) is melted to be bonded (welded) to the outer cover 3.

Since such an outer cover 3 is exposed to chemicals such as various kinds of disinfectants or high temperatures and pressures during repeated disinfection or sterilization treatments, it is required for the outer cover 3 to have chemical resistance and heat resistance. In addition, it is also required that the outer cover 3 is mainly made of a material having flexibility to prevent damage resulting from friction to tissue in the body cavity.

The inventors of this invention have made extensive research, and as a result, they have found that the outer cover 3 is formed into a laminated structure comprised of a first layer and a second layer which forms an outermost layer of the laminated structure, in which the first layer is formed of a material containing as a major component thereof polyolefin-based thermoplastic elastomer (hereinafter, also referred to as "TPO") or styrene-ethylene · butylene-styrene block copolymer (hereinafter, also referred to as "SEBS"), and the second layer is formed of a material containing as a major component thereof polyolefin, and this structure makes it possible for the outer cover 3 to have sufficient flexibility and to exhibit especially excellent heat resistance, chemical resistance and abrasion resistance, leading to the completion of the present invention.

As shown in Fig. 2, in the flexible insertion tube 1 of this embodiment, the outer cover 3 is formed into a laminated structure comprised of an inner layer (first layer) 31 and an outermost layer (second layer) 32, in which the inner layer 31 is formed of a material containing TPO or SEBS and the outer layer 32 is formed of a material containing polyolefin (note that, in this embodiment, the outermost layer is also referred simply as "outer layer". In this embodiment, the inner layer 31 formed of a material containing TPO or SEES and the outermost layer 32 formed of a material containing polyolefin are arranged adjacent to each other and the TPO or SEBS has excellent affinity with the polyolefin, so that adhesion between the inner layer 31 and the outermost layer 32 can be made excellent, and as a result, it is possible to obtain a more stable outer cover.

The amount of TPO or SEBS contained in the constituent material of the inner layer 31 is not limited to any specific value, but it is preferred that the outer cover 3 is formed of a material containing TPO or SEBS as a major component thereof.

Specifically, when the TPO is used as the constituent material of the inner layer 31, heat resistance is especially improved. When the TPO is used as the constituent material of the inner layer 31, the amount of TPO contained in the constituent material is preferably 50 wt% or more, more preferably 60 wt% or more, and even more preferably 70 to 100 wt%.

Further, when the SEES is used as the constituent material of the inner layer 31, chemical resistance is especially improved. When the SEBS is used as the constituent material of the inner layer 31, the amount of SEBS contained in the constituent material is preferably 50 wt% or more, more preferably 60 wt% or more, and even more preferably 70 to 100 wt%.

On the other hand, when both the TPO and SEBS are used as the constituent material of the inner layer 31, both of heat resistance and chemical resistance are conspicuously improved due to the synergistic effect derived from the materials. When both the TPO and SEBS are used as the constituent material of the inner layer 31, the amount of the TPO and SEBS contained in the constituent material is preferably 50 wt% or more, more preferably 60 wt% or more, and even more preferably 70 to 100 wt%.

Further, when a material containing the SEBS is used as the constituent material of the inner layer 31, flexibility is further improved.

Generally, TPO is comprised of a hard segment and a soft segment. Examples of a material that can constitute a hard segment include polyethylene, polypropylene and the like. The hard segment may be constituted from one or more of these materials. Further, examples of a material that can constitute a soft segment include vulcanized ethylene-propylene-diene copolymer rubber (EPDM), butyl rubber, chloroprene rubber and the like. The soft segment may be constituted from one or more of these materials.

The average thickness of the inner layer 31 (excluding those portions corresponding to the protruding portions 4) is not limited to any specific value, but is preferably in the range of about 0.05 to 0.8 mm, more preferably in the range of about 0.05 to 0.4 mm.

As described above, both the TPO and SEBS have excellent affinity (miscibility) with polyolefin which is contained in the constituent material of the outer layer (outermost layer) 32, so that the adhesion between the inner layer and the outer layer 32 can be made higher.

Further, the constituent material of the inner layer 31 may contain polyolefin as described later. When the constituent material of the inner layer 31 contains polyolefin, the adhesion between the inner layer 31 and the outermost layer 32 can be further improved, and the entire of the outer cover 3 can have an appropriate resiliency or elasticity.

When the constituent material of the inner layer 31 contains polyolefin, the amount of the polyolefin is preferably 5 to 70 parts by weight with respect to 100 parts by weight of TPO or SEBS, and more preferably 10 to 50 parts by weight. This makes it possible to exhibit the effects described above more conspicuously.

Examples of polyolefin which constitutes the outermost layer 32 include polyethylene, polypropylene, and the like, but are not limited thereto. Among these polyolefins, polypropylene is preferable. The use of polypropylene makes it possible for the flexible insertion tube 1 to have more excellent abrasion resistance with maintaining the excellent chemical resistance and heat resistance.

Such polypropylenes can be roughly divided into three types which include homopolypropylene, random polypropylene, and block polypropylene. Among them, homopolypropylene is particularly preferable. The use of homopolypropylene makes it possible for the flexible insertion tube 1 to further improve heat resistance with maintaining the excellent abrasion resistance.

In this regard, it is to be noted that, besides polyolefin, there are know various polymeric materials having relatively high abrasion resistance. However, it is not possible to obtain such an effect as the present invention even if an outer cover made of any of such other polymeric materials is used together with an inner cover made of a constituent material containing TPO or SEBS. For example, as for such a polymeric material having high abrasion resistance, polyester and polystyrene can be mentioned. However, when such a material is used, there arises a problem in that adhesion between the inner layer 31 and the outer layer 32 will be lowered and chemical resistance and heat resistance are lowered.

The amount of the polyolefin contained in the constituent material of the outermost layer 32 is not limited to a specific value, but it is preferred that the outermost layer 32 is mainly constituted from polyolefin. The amount of the polyolefin in the constituent material is preferably 50wt% or more and more preferably 70wt% or more. This makes it possible to exhibit more excellent abrasion resistance.

As described above, the constituent material of the outermost layer 32 may further contains other material besides the polyolefin. In this case, it is preferable to contain thermoplastic elastomer. This makes it possible for the entier of the outer cover 3 to exhibit especially excellent flexibility (elasticity).

No limitation is imposed on the kind of the thermoplastic elastomer, but it is preferable to use TPO or SEBS as described above. These thermoplastic elastomers have excellent affinity (miscibility) with polyolefin, it is possible to form a stable outermost layer 32, and the adhesion between the outermost layer 32 and the inner layer 31 is further improved.

In this regard, the amount of the thermoplastic elastomer contained in the constituent material of the outermost layer 32 is preferably 70 parts by weight or less with respect to 100 parts by weight of polyolefin, and more preferably 50 parts by weight or less. This makes it possible to exhibit the effects described above more conspicuously. On the other hand, if the amount of the thermoplastic elastomer exceeds the above upper limit value, there is a case that sufficient abrasion resistance cannot be obtained.

The average thickness of the outermost layer 32 is not limited to any specific value, but it is preferably in the range of about 0.01 to 0.5 mm, and more preferably in the range of about 0.03 to 0.3 mm. If the thickness of the outermost layer 32 is too thin, there is a case that sufficient abrasion resistance cannot be obtained depending on the amount or kind of the polyolefin. On the other hand, the thickness of the outermost layer is too thick, there is a case that the flexible insertion tube 1 cannot be freely bent depending on the amount or kind of the polyolefin.

Further, the outermost layer 32 may have a portion in which the thickness of the outermost layer 32 changes along the longitudinal direction thereof, but it is preferred that the outermost layer 32 has a uniform thickness along the direction. This makes it possible to further improve operability when the flexible insertion tube 1 is inserted into a body cavity, thus reducing the burden for a patient.

When the average thickness of the above-described inner layer (first layer) 31 is defined as a (mm) and the average thickness of the outermost layer (second layer) 32 is defined as b (mm), it is preferred that the relationship represented by the equation of 0.25 ≤ b/a ≤ 1.0 is satisfied , and it is more preferable that the relationship represented by the equation of 0.1 ≤ b/a ≤ 0.5 is satisfied. By satisfying such a relationship, it is possible to provide a flexible insertion tube 1 having excellent chemical resistance, heat resistance and abrasion resistance with maintaining the sufficient flexibility. On the other hand, if the value b/a is less than the lower limit value, there is a case that sufficient abrasion resistance cannot be obtained depending on the amount or kind of the polyolefin. On the other hand, if the value b/a exceeds the above upper limit value, there is a case that sufficient flexibility cannot be obtained depending on the amount or kind of the polyolefin.

As described above, the flexible insertion tube 1 of this embodiment (a flexible tube for an endoscope according to the present invention) includes the outer cover 3 which is comprised of the inner layer (first layer) 31 formed of a material containing as a major component thereof TPO or SEBS and the outermost layer (second layer) 32 formed of a material containing polyolefin. In this regard, however, it is to be noted that in the case where the outer cover 3 includes an outermost layer 32 formed of a material containing polyolefin but does not include an inner layer 31 formed of a material containing as a major component thereof TPO or SEBS, namely in the case where the outer cover 3 does not have a layer corresponding to the first layer of the present invention or in the case where the outer cover 3 does not have a layer corresponding to the second layer of the present invention, it is not possible to obtain the effects of the present invention.

For example, in the case where the outer cover 3 does not have a layer corresponding to the first layer, sufficient flexibility cannot be obtained. Further, in the case where the outer cover 3 does not have a layer corresponding to the second layer, sufficient abrasion resistance cannot be obtained.

Further, in the case where the outer cover 3 is formed from a single layer made of a material containing a mixture of the material for the first layer (that is, TPO or SEBS) and the material for the second layer (that is, polyolefin) without providing separate layers such as the first and second layers like the present invention, it is not possible to obtain sufficient abrasion resistance.

The average thickness of the outer cover 3 comprised of the inner layer 31 and the outermost layer 32 (excluding those portions corresponding to the protruding portions 4) is not limited to a specific value so long as the core member 2 and the elongated members passing through the inside of the core member 2 are protected from fluids such as body fluids and so long as the bendability of the flexible insertion tube 1 is not impaired. Specifically, the average thickness of the outer cover 3 is preferably in the range of about 0.08 to 0.9 mm, more preferably in the range of about 0.1 to 0.8 mm, even more preferably in the range of about 0.3 to 0.5 mm.

Further, the thickness of the outer cover 3 may be varied at different portions along the longitudinal direction thereof, but is preferably substantially uniform. A substantially uniform thickness of the outer cover 3 improves the operability of the flexible insertion tube 1 at the time when the flexible insertion tube 1 is inserted into the body cavity, and thereby a burden placed on a patient can be further reduced.

When necessary, various additives may be added to (contained to) the material of the outer cover 3 (hereinafter also referred to as an "outer cover material"). Examples of the additives include plasticizer; inorganic filler; pigment; various stabilizers (such as antioxidant, photostabilizer, antistatic agent, blocking inhibitor, and lubricant); X-ray contrast medium; and the like.

The flexible insertion tube 1 having the outer cover of the laminated structure as described above can be continuously manufactured by, for example, covering the outer periphery of the core member 2 with the outer cover materials of the inner layer and the outermost layer simultaneously using an extrusion molding machine having two or more coaxial extrusion openings (orifices).

Further, by adjusting the discharge quantity (extrusion quantity) of the outer cover materials for the respective layers to be extruded through the extrusion openings and the pulling speed of the core member 2, it is possible to control the thickness of each layer of the outer cover 3.

Alternatively, the flexible insertion tube 1 may be manufactured in the following manner. For example, the outer cover 3 is formed as a hollow tubular body (tube), and then the core member 2 is inserted into the thus obtained outer cover 3, whereafter, a heating process or the like is carried out to bond the outer cover 3 to the core member 2.

The temperature of the material during extrusion molding is not limited to any specific value, but is preferably in the range of about 130 to 220°C, more preferably in the range of about 165 to 205°C. By setting the temperature of the material during extrusion molding to a value within the above range, the moldability of the outer cover material at the time when the outer cover 3 is formed becomes excellent, and thereby the outer cover 3 can have more uniform thickness. In this regard, it is to be noted that the temperature of the material may be changed for each of the layers.

Next, another embodiment of the flexible insertion tube (flexible tube for an endoscope according to the present invention) will be described.

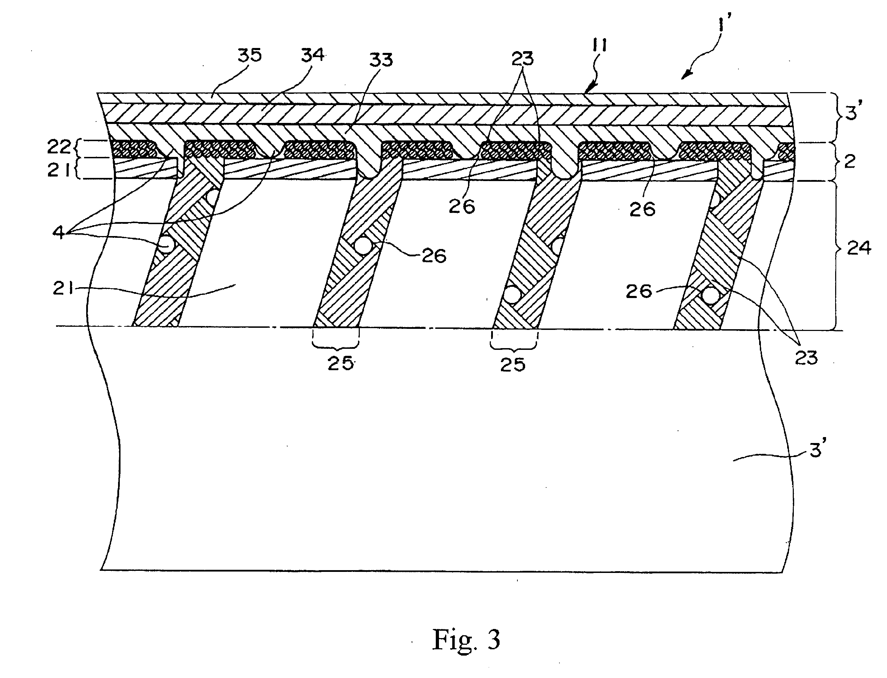

Fig. 3 is a longitudinal sectional view which shows another embodiment of the flexible insertion tube (flexible tube for an endoscope according to the present invention).

Hereinbelow, a flexible insertion tube 1' shown in Fig. 3 will be described by focusing on elements that are different from those described above for the flexible insertion tube 1, and therefore a description of the same elements will be omitted.

### <Flexible insertion tube 1'>

The flexible insertion tube 1' is the same as the flexible insertion tube 1 described above except that an outer cover 3' of the flexible insertion tube 1' is formed into a laminated structure which includes an inner layer 33, an intermediate layer (first layer) 34 and an outermost layer (second layer) 35.

In the following, a description will be made with regard to an example in which the intermediate layer (first layer) 34 is made of a material containing as a major component thereof TPO or SEBS, and the outermost layer (second layer) 35 is made of a material containing as a major component thereof polyolefin. The structure of the intermediate layer 34 (its material, shape and thickness and the like) is the same as that of the inner layer 31 of the first embodiment, and the structure of the outermost layer 35 (its material, shape and thickness and the like) is the same as that of the outermost layer 32 of the first embodiment.

As described above, in the case where the outer cover 3 is formed into the laminated structure comprised of three or more layers, it is preferred that the layer formed of a material containing TPO or SEBS (that is, the intermediate layer 34) is arranged adjacent to or in contact with the layer containing polyolefin (that is, the outermost layer 35). According to this structure, since TPO or SEBS has excellent affinity with polyolefin, the adhesion between the intermediate layer 34 and the outermost layer 35 can be made excellent, and as a result thereof, it is possible to obtain a more stable outer cover 3.

Examples of the constituent material of the inner layer 33 include various resins having flexibility such as polyolefin (e.g., polyvinyl chloride, polyethylene, polypropylene, ethylene-vinylacetate copolymer, and the like), polyamide, polyester (e.g., polyethylene terephthalate (PET), polybutylene terephthalate, and the like), polyurethane, polystyrene resin, fluorine-based resin (e.g., polytetrafluoroethylene, ethylene-tetrafluoroethylene copolymer, and the like), and polyimide; and various elastomers such as polyurethane-based thermoplastic elastomer, polyester-based thermoplastic elastomer, polyolefin-based thermoplastic elastomer, polyamide-based thermoplastic elastomer, polystyrene-based thermoplastic elastomer, fluorine-based thermoplastic elastomer, silicone rubber, fluorine rubber, latex rubber; and the like, but are not limited thereto. These constituent materials can be used singly or in combination of two or more. Among them, polyurethane-based thermoplastic elastomer, polyolefin-based thermoplastic elastomer, or polyester-based thermoplastic elastomer is particularly preferable. The use of such a material makes it possible to control the formation of the protruding portions 4 easily. Further, it is also possible to improve the adhesion between the inner layer 33 and the intermediate layer 34.

The average thickness of the inner layer 33 (excluding those portions corresponding to the protruding portions 4) is not limited to any specific value, but is preferably in the range of about 0.03 to 0.8 mm, more preferably in the range of about 0.03 to 0.4 mm.

As described above, by forming the outer cover 3' from the intermediate layer 34 having excellent chemical resistance and heat resistance, the outermost layer 35 having excellent abrasion resistance, and the inner layer 33 which can provide excellent adhesion with respect to the core member 2, each of the layers can exhibit its function sufficiently. As a result, it is possible to obtain an outer cover 3' having especially excellent properties due to the synergistic effect derived from the functions.

Consequently, the flexible insertion tube 1' having the outer cover 31 described above and the electronic endoscope equipped with the flexible insertion tube 1' can have the same effects.

In this regard, it is to be noted that the structure of the outer cover 3' is not limited to that shown in the drawing, and the outer cover 3' may be formed into a laminated structure having four or five or more layers.

Further, as described above, in this embodiment, the intermediate layer 34 is formed of a material containing TPO or SEBS. However, the present invention is not limited to this structure. For example, the inner layer 33 may be formed of a material containing TPO or SEBS, and the intermediate layer 34 may be formed of a material other than those mentioned above. In this case, it is preferred that the intermediate layer 34 is formed into a layer which is more flexible or elastic than the outermost layer 35. This makes it possible for the intermediate layer 34 to exhibit a cushioning function between the inner layer 33 and the outermost layer 35.

Although the flexible tube for an endoscope and the endoscope according to the present invention have been described, the present invention is not limited thereto, and so long as the same functions are achieved, it is possible to make various changes or additions to each element (portion) thereof.

In each of the embodiments described above, a flexible insertion tube has been described as a representative of flexible tubes for endoscopes, but it goes without saying that the flexible tube for an endoscope of the present invention can be applied to flexible connection tubes. Further, although an electronic endoscope (electronic scope) has been described as a representative of endoscopes, it goes without saying that the flexible tube for an endoscope and the endoscope of the present invention can be applied to optical endoscopes (endoscopes of fiber scope type).

Further, in each of the embodiments described above, the description was made based on the case where a flexible tube for an endoscope of the present invention is applied to an endoscope for medical use, but the flexible tube for an endoscope of the present invention can be applied to endoscopes for industrial use.

### Examples

Next, specific embodiments of the present invention will be described.

### 1. Manufacture of flexible tube for an endoscope

### (Example 1)

First, a spiral coil having an outer diameter of 9 mm and an inner diameter of 7 mm was prepared by winding a band-shaped stainless steel member having a width of 3.2 mm. Then, the spiral coil was jointed at one end thereof to nodal rings. Next, a plurality of bundles each having ten fine wires made of stainless steel (each of the fine wires had a diameter of 0.08 mm) were prepared, and then these bundles were braided to obtain a reticular tube. The outer periphery of the spiral coil with the nodal rings was covered with this reticular tube to obtain a core member.

Next, polyolef in-based thermoplastic elastomer (TPO) and polyolefin (homopolypropylene) were prepared. The polyolefin-based thermoplastic elastomer was in the form of a block copolymer which was comprised of a hard segment constituted from polypropylene and a soft segment constituted from ethylene-propylene-diene copolymer. Then, the outer periphery of the core member was covered with an outer cover having a laminated structure comprised of an inner layer formed of TPO and an outermost layer formed of polyolefin by using the extrusion molding method to obtain a flexible tube for an endoscope. In thus obtained flexible tube, the average thickness of the outer cover was 0.45 mm, in which the average thickness of the inner layer was 0.4 mm and the average thickness of the outermost layer was 0.05 mm, and the length of the flexible insertion tube was 2 m.

In this Example, the outer cover having the laminated structure was formed using an extrusion molding machine having two coaxial extrusion openings. Namely, the outer cover having the laminated structure was continuously manufactured by extruding the inner and outermost layers simultaneously so as to cover the core member with the outer cover. In this case, the temperature of the material at the extrusion molding was 190°C.

### (Examples 2 and 3)

In each of these Examples, a flexible tube for an endoscope was manufactured in the same manner as in Example 1 except that the average thickness of each of the inner layer and the outermost layer and the constituent material of the inner layer were changed as shown in Table 1.

### (Examples 4 to 6)

In each of these Examples, a flexible tube for an endoscope was manufactured in the same manner as in Example 1 except that styrene-ethylene · butylene-styrene block copolymer (SEES) was used as the constituent material of the inner layer, and the average thickness of each of the inner and outermost layers and the constituent material of the inner layer were changed as shown in Table 1. In this case, the temperature of the material at the extrusion molding was 190°C.

### (Examples 7 and 8)

In each of these Examples, a flexible tube for an endoscope was manufactured in the same manner as in Example 1 except that a material containing polyolefin and TPO was used as the constituent material of the outermost layer, and the compounding ratio of these materials and the average thickness of the outermost layer were changed as shown in Table 1.

### (Examples 9 and 10)

In each Example, a flexible tube for an endoscope was manufactured in the same manner as in Example 4 except that a material containing polyolefin and SEBS was used as the constituent material of the outermost layer, and the compounding ratio of these materials, the average thickness of the outermost layer and the constituent material of the inner layer were changed as shown in Table 1.

### (Example 11)

A flexible tube for an endoscope was manufactured in the same manner as in Example 1 except that a mixture containing 50 parts by weight of TPO and 50 parts by weight of SEBS was used as the constituent material of the inner layer.

### (Example 12)

A flexible tube for an endoscope was manufactured in the same manner as in Example 1 except that the outer cover was formed into a laminated structure comprised of an inner layer, an intermediate layer and an outermost layer, in which the inner layer was formed of polyurethane-based thermoplastic elastomer (manufactured and sold by DIC Bayer Polymer Ltd. with the product name of "PANDEX"), the outermost layer was formed of polyolefin, and the intermediate layer was formed of TPO.

In this Example, the outer cover having the laminated structure was manufactured using an extrusion molding machine provided with three coaxial extrusion openings. Namely, the outer cover having the laminated structure was continuously manufactured by extruding the inner, intermediate and outermost layers simultaneously so as to cover the core member with the outer cover.

In this case, the average thickness of each of the inner, intermediate and outermost layers were 0.2 mm, 0.2 mm and 0.05 mm, respectively. Further, the temperature of the material at the extrusion molding was 190°C.

### (Example 13)

A flexible tube for an endoscope was manufactured in the same manner as in Example 12 except that SEBS was used as the constituent material of the intermediate layer, and the average thickness of each of the inner, intermediate and outermost layers was changed as shown in Table 1. In this case, the temperature of the material at the extrusion molding was also 190°C.

### (Comparative Example 1)

A flexible tube for an endoscope was manufactured in the same manner as in Example 1 except that an outer cover formed of polyurethane-based thermoplastic elastomer (manufactured and sold by DIC Bayer Polymer Ltd. with the product name of "PANDEX" ) was formed onto the outer periphery of the core member by means of extrusion molding.

### (Comparative Example 2)

A flexible tube for an endoscope was manufactured in the same manner as in Example 1 except that polyurethane-based thermoplastic elastomer (manufactured and sold by DIC Bayer Polymer Ltd. with the product name of "PANDEX") was used as the constituent material of the inner layer.

### (Comparative Example 3)

A flexible tube for an endoscope was manufactured in the same manner as in Comparative Example 1 except that the outer cover was formed of only polyolefin.

### (Comparative Example 4)

A flexible tube for an endoscope was manufactured in the same manner as in Comparative Example 1 except that the outer cover was formed of a mixture of polyolefin and TPO. In this case, the compounding ratio of polyolefin and TPO was 100 : 70 in weight ratio.

### (Comparative Example 5)

A flexible tube for an endoscope was manufactured in the same manner as in Comparative Example 1 except that the outer cover was formed of TPO.

### (Comparative Example 6)

A flexible tube for an endoscope was manufactured in the same manner as in Comparative Example 1 except that the outer cover was formed of SEBS.

The constituent material of the outer cover, the constituent material of each of the layers and the average thickness of the outer cover of each of Examples and Comparative Examples are shown in Table 1.

Further, it should be noted that polyolefin-based thermoplastic elastomer, styrene-ethylene- butylene-styrene block copolymer, polyolefin, and polyurethane-based elastomer are abbreviated as "TPO", "SEBS", "PO" and "TPU" in Table 1, respectively.

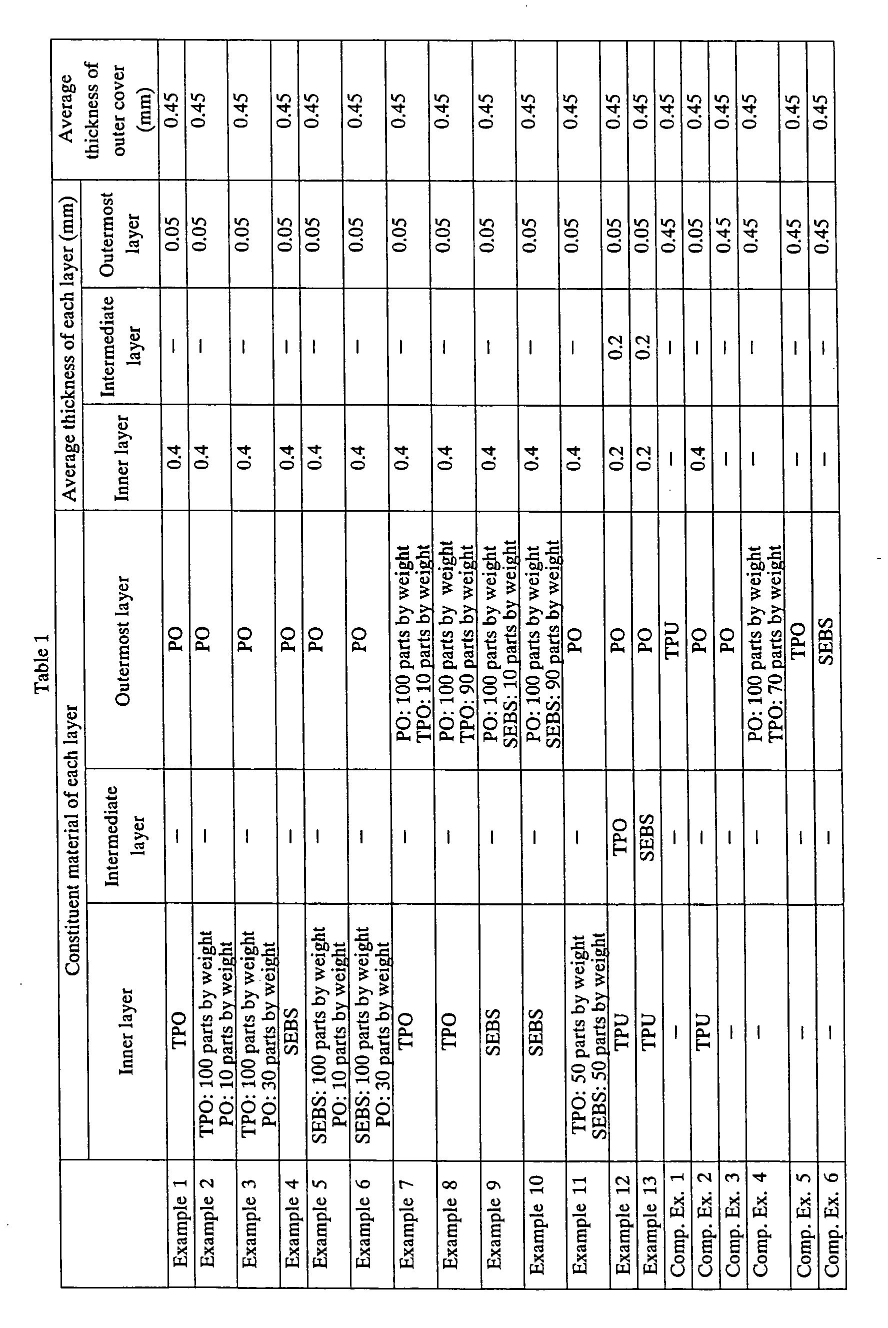

### 2. Evaluation of properties of flexible tube for an endoscope

### 2.1 Chemical resistance test

Chemical resistance test was carried out on the flexible tube for an endoscope manufactured in each of Examples and Comparative Examples in the following manner.

In this chemical resistance test, each of the flexible tubes for endoscopes was subjected to a cleaning and disinfection operation by the use of a heating-type cleaner (manufactured and sold by BHT Corp. with the product name of "SME 2000").

This cleaning and disinfection operation was carried out through four processes, i.e., a cleaning process using a cleaning solution (nonionic surfactant)(about 10 minutes), a disinfection process using a disinfectant solution (about 0.24 wt% aqueous glutaraldehyde solution) (about 10 minutes), a washing process using hot water at about 60°C (about 10 minutes), and a drying process with hot air (about 5 minutes).

After this cleaning and disinfection operation was repeated 2,000 times, the appearance of the flexible tube for an endoscope was observed, and was then evaluated according to the following four criteria.
A: No change in appearance
B: Almost no loss of luster and yellowing
C: Slight loss of luster and yellowing
D: Noticeable loss of luster and yellowing

### 2.2 Heat resistance test

Heat resistance test was carried out on the flexible tube for an endoscope manufactured in each of Examples and Comparative Examples in the following manner.

Three flexible tubes for endoscopes were prepared for each of Examples and Comparative Examples. Each of the flexible tubes for endoscopes was subjected to autoclave sterilization for 15 minutes at a temperature of 135°C and under a pressure of 2.2 atmospheres, and was then rapidly cooled in ice water. This series of operations was repeated 20 times.

After the series of operations was repeated 20 times, the degree of deterioration of each of the flexible tubes for endoscopes, especially the degree of loss (lowering) of flexibility was examined, and was then evaluated according to the following four criteria.
A: Almost no change in flexibility
B: Slight loss of flexibility
C: Noticeable loss of flexibility
D: Stiff state (significant deterioration)

### 2.3 Abrasion resistance test

Abrasion resistance test was carried out on the flexible tube for an endoscope manufactured in each of Examples and Comparative Examples in accordance with SO9211-4. In more details, the abrasion resistance test was carried out under the following conditions.

In the flexible tube for an endoscope of each of Examples and Comparative Examples, the surface of the outer cover was rubbed using an abrasion resistance test machine for 50 cycles with a power of 5N. In this test, as for the abrasive head of the abrasion resistance test machine, a cotton cheesecloth was used.

After the series of operations was carried out, the degree of abrasion on the surface of the outer cover of each of the flexible tubes for endoscopes, and was then evaluated according to the following four criteria.
A: Almost no scratch was observed on the surface of the outer cover
B: Scratch was observed slightly on the surface of the outer cover
C: Scratch was observed on the surface of the outer cover
D: Noticeable scratch was observed on the surface of the outer cover

### 2.4 Resilience test

Resilience test was carried out on the flexible tube for an endoscope manufactured in each of Examples and Comparative Examples in the following manner.

In this resilience test, ten flexible tubes for endoscopes were prepared for each of Examples and Comparative Examples, and then these ten flexible tubes for endoscopes were tied in a bundle to examine whether or not this bundle could be bent.

After such bending operation was carried out for the bundled ten flexible tubes, the resilience of each of the flexible tubes for endoscopes was evaluated according to the following four criteria.
A: Extremely good resilience
B: Good resilience
C: Poor resilience
D: Virtually no resilience (stiff state)

The results of these evaluations are shown in Table 2.

**Table 2**

| | Chemical Resistance | Heat resistance | Abrasion resistance | Resilience |
|---|---|---|---|---|
| Example 1 | A | A | A | B |
| Example 2 | A | A | A | B |
| Example 3 | A | A | A | A |
| Example 4 | A | A | A | B |
| Example 5 | A | A | A | B |
| Example 6 | A | A | B | A |
| Example 7 | A | A | B | B |
| Example 8 | A | A | C | B |
| Example 9 | A | A | B | B |
| Example 10 | A | A | C | B |
| Example 11 | A | A | A | B |
| Example 12 | A | A | A | A |
| Example 13 | A | A | A | A |
| Comp. Ex. 1 | D | D | A | A |
| Comp. Ex. 2 | B | D | A | B |
| Comp. Ex. 3 | A | A | A | D |
| Comp. Ex. 4 | A | A | C | D |
| Comp. Ex. 5 | A | A | D | D |
| Comp. Ex. 6 | A | A | D | D |

As is apparent from Table 2, all of the flexible tubes for endoscopes of the present invention had excellent heat resistance, chemical resistance and abrasion resistance with maintaining sufficient flexiblity.

Further, each of the flexible tubes for endoscopes which has the outer cover having the laminated structure comprised of the three layers exhibited the functions of the respective layers sufficiently, and due to the synergistic effect derived from the functions, these flexible tubes for endoscopes had especially excellent operability.

In contrast, each of the flexible tubes for endoscopes of Comparative Examples has poor performances as compared with the flexible tubes for endoscopes of Examples. In particular, the flexible tube for an endoscope of Comparative Example 3 of which outer cover was formed of only polyolefin had poor flexibility.

### Effects of the Invention

As has been described above, according to the present invention, it is possible to obtain a flexible tube for an endoscope having excellent heat resistance, chemical resistance and abrasion resistance with maintaining sufficient flexibility.

Finally, it is to be understood that many changes and additions may be made to the embodiments described above without departing from the scope and spirit of the invention as defined in the following claims.

Further, it is also to be understood that the present disclosure relates to subject matter contained in Japanese Patent Application No. 2003-193086 (filed on July 7, 2003) which is expressly incorporated herein by reference in its entirety.

## Claims

1. A flexible tube for an endoscope, comprising:
a tubular core member; and
an outer cover provided around the core member, the outer cover having a laminated structure comprised of a first layer and a second layer which forms an outermost layer of the laminated structure, the first layer being formed of a material containing as a major component thereof polyolefin-based thermoplastic elastomer or styrene-ethylene · butylene-styrene block copolymer, and the second layer being formed of a material containing as a major component thereof polyolefin.

2. The flexible tube for an endoscope as claimed in claim 1, wherein the first layer and the second layer are arranged adjacent to each other.

3. The flexible tube for an endoscope as claimed in claim 1, wherein the material of the second layer further contains thermoplastic elastomer.

4. The flexible tube for an endoscope as claimed in claim 3, wherein the amount of the thermoplastic elastomer contained in the material of the second layer is equal to or less than 70 parts by weight with respect to 100 parts by weight of the polyolefin.

5. The flexible tube for an endoscope as claimed in claim 1, wherein the average thickness of the second layer is in the range of 0.01 to 0.5mm.

6. The flexible tube for an endoscope as claimed in claim 1, wherein the polyolefin is mainly constituted from polypropylene.

7. The flexible tube for an endoscope as claimed in claim 1, wherein the amount of the polyolefin-based thermoplastic elastomer or styrene-ethylene · butylene-styrene block copolymer contained in the material of the first layer is 50wt% or more.

8. The flexible tube for an endoscope as claimed in claim 1, wherein the material of the first layer contains both the polyolefin-based thermoplastic elastomer and the styrene- ethylene · butylene-styrene block copolymer, wherein the amount of the polyolefin-based thermoplastic elastomer and the styrene-ethylene · butylene-styrene block copolymer contained in the material of the first layer is 50wt% or more.

9. The flexible tube for an endoscope as claimed in claim 1, wherein the average thickness of the outer cover is in the range of 0.08 to 0.9mm.

10. The flexible tube for an endoscope as claimed in claim 1, wherein when the average thickness of the first layer is defined as a (mm) , and the average thickness of the second layer is defined as b (mm), the relationship represented by the equation of 0.25 ≤ b/a ≤ 1.0 is satisfied.

11. An endoscope equipped with the flexible tube defined in any one of claims 1 to 10.
